# EUROPEAN PATENT APPLICATION

(11) **EP 4 850 665 A1**
(43) Date of publication of application: **09.12.2026**
(21) Application number: 25827135.2
(22) Date of filing: 31.01.2025
(51) Int. Cl.: A61K 8/73, A61K 8/9794, A61K 8/06, A61K 8/02, A61K 8/04, A61Q 19/00, A61Q 17/04

(54) **OIL-IN-WATER (O/W) COSMETIC COMPOSITION COMPRISING AGAROSE**

(30) Priority: 26.06.2024 KR 20240083467
(71) Applicant: Kolmar Korea Co., Ltd., Sejong 30004 (KR)
(72) Inventor: OH, Seong Min, Seoul 06800 (KR)
(74) Representative: Nederlandsch Octrooibureau
(86) International application number: PCT/KR2025/001561
(87) International publication number: WO 2026/005170

(57) **Abstract**

An oil-in-water (O/W) cosmetic composition of the present invention can exhibit excellent elasticity and sufficient hardness by optimal combination of an elastic thickener including agarose and an auxiliary thickener, and thus is suitable for molding into a stick shape or the like. Therefore, the cosmetic composition can offer an excellent sensory feel, and does not collapse even during application, enabling convenient use.

## Description

### TECHNICAL FIELD

The present invention relates to an oil-in-water (O/W) cosmetic composition comprising agarose, and more particularly, to a cosmetic composition of an oil-in-water (O/W) formulation, which has sufficient hardness to be suitable for forming into a stick formulation and has a unique sensory feel, by optimally combining agarose and an auxiliary thickener.

### BACKGROUND ART

Stick-type cosmetics are easy to carry and convenient to apply without the need for a separate applicator, and thus correspond to the most widely used type of cosmetics regardless of age or gender. In general, stick-type cosmetics are at risk of breaking when rubbed on the skin, and thus are required to have sufficient hardness formed. Accordingly, cosmetic compositions used in stick-type cosmetics employ an oil-dispersion formulation or a water-in-oil formulation suitable for forming hardness.

Unlike an oil-dispersion formulation or a water-in-oil formulation, a composition of an oil-in-water formulation has a low oily feel, and thus can provide a refreshing and cool sensation, and in particular, has the advantage of being usable on oily skin or acne-prone skin due to its low oil content. However, not only is it difficult to form the composition into a stick form, but even when the stick form is achieved, there is also a technical difficulty in forming hardness suitable for rubbing on the skin.

Accordingly, there is a need to develop a composition of an oil-in-water formulation having sufficient hardness to be formed into a stick formulation. If the advantages of stick-type convenience can be stably applied to a cosmetic composition of an oil-in-water formulation as well, people with sensitive skin types such as oily skin will also be able to conveniently use stick-type cosmetics, and since a greater variety of functional cosmetics can be manufactured in a stick-type, consumers will have a wider range of product choices according to their needs.

Accordingly, the present inventors have completed the present invention by using agarose as an elastic thickener and optimally combining an auxiliary thickener therewith, thereby achieving a cosmetic composition of an oil-in-water (O/W) formulation, which has hardness suitable for being formed into a stick formulation and has an excellent sensory feel due to its unique elasticity.

### DETAILED DESCRIPTION OF THE INVENTION

### TECHNICAL PROBLEM

An object of the present invention is to provide a cosmetic composition of an oil-in-water (O/W) formulation, which has sufficient hardness to be suitable for forming into a stick formulation and has a unique sensory feel.

The technical problems of the present invention are not limited to the technical problems mentioned above, and other technical problems not mentioned will be clearly understood by those skilled in the art from the description below.

### TECHNICAL SOLUTION

According to an embodiment of the present invention, there is provided an oil-in-water (O/W) cosmetic composition including an elastic thickener and an auxiliary thickener, wherein the elastic thickener includes agarose.

### ADVANTAGEOUS EFFECTS

The oil-in-water cosmetic composition of the present invention can form sufficient hardness, and thus is suitable for forming into a stick-type or the like, and can maintain a stable formulation without collapsing during application to the skin.

In addition, the cosmetic composition has excellent elasticity, and thus can provide an excellent sensory feel due to elasticity.

In addition, the cosmetic composition has a low water separation rate, and thus can have excellent long-term storage stability, thereby having excellent marketability.

In addition, the cosmetic composition has a low melting temperature, and thus flowability can be easily increased, so that the cosmetic composition may be suitable for forming into a desired form.

### DESCRIPTION OF THE DRAWINGS

To help understand the drawings in the detailed description of the present invention more sufficiently, a brief description of each drawing is provided.

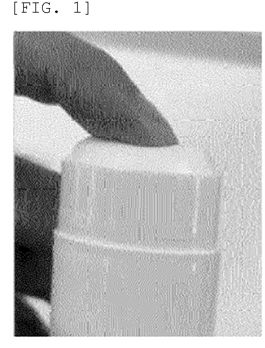

FIG. 1 is a photograph showing Example 1.

### MODES OF THE INVENTION

According to an embodiment of the present invention, there is provided an oil-in-water (O/W) cosmetic composition including an elastic thickener and an auxiliary thickener, wherein the elastic thickener includes agarose.

In addition, the auxiliary thickener may include carrageenan and konjac root extract (Amorphophallus Konjac Root Extract).

In addition, the agarose may be included in an amount of 0.7 to 2.0% by weight based on the total weight of the oil-in-water cosmetic composition.

In addition, the carrageenan may be included in an amount of 0.05 to 0.4% by weight based on the total weight of the oil-in-water cosmetic composition.

In addition, the konjac root extract may be included in an amount of 0.05 to 0.15% by weight based on the total weight of the oil-in-water cosmetic composition.

In addition, the oil-in-water cosmetic composition may further include a UV-blocking agent.

In addition, the oil-in-water cosmetic composition may be semi-solid.

In addition, the oil-in-water cosmetic composition may be a gel formulation having elasticity.

In addition, the oil-in-water cosmetic composition may be a stick-type cosmetic composition.

Unless defined otherwise, all technical and scientific terms used herein have the same meaning as commonly understood by one of ordinary skill in the art to which the present invention pertains. In general, the nomenclature used herein is well known and commonly used in the art. Furthermore, in describing embodiments of the present invention, when it is determined that a detailed description of a related known configuration or function would hinder the understanding of the embodiments of the present invention, the detailed description thereof will be omitted. Furthermore, embodiments of the present invention will be described below, but the technical spirit of the present invention is not limited or restricted thereto, and may be modified and implemented in various ways by those skilled in the art.

When a part of this specification is said to include a certain component, unless specifically stated to the contrary, this means that other components are not excluded, but may be further included. In this specification, the term "and/or" includes a combination of a plurality of related items or any one item among a plurality of related items.

According to an embodiment of the present invention, there is provided an oil-in-water (O/W) cosmetic composition including an elastic thickener and an auxiliary thickener, wherein the elastic thickener includes agarose. The oil-in-water cosmetic composition according to an embodiment of the present invention has sufficient hardness and has a low melting temperature, so that flowability thereof is easy to control, and thus the oil-in-water cosmetic composition may be very suitable for forming into a stick shape or the like. In addition, the oil-in-water cosmetic composition, due to its unique elasticity, when applied to the skin, may provide a user with excellent sensory feel, including optimal spreadability resulting from excellent elasticity.

Agarose is a natural polysaccharide extractable from seaweed, and is a linear polymer in chain form composed of disaccharide units including D-galactose and 3,6-anhydro-L-galactose. Agarose in chain form can form a double helix structure through hydrogen bonding, and is stabilized by hydrogen bonding and van der Waals forces. Specifically, in the present invention, agarose, as an elastic thickener, can impart elasticity to the oil-in-water cosmetic composition. In addition, agarose chains can form a gel formulation having a three-dimensional network structure, thereby serving to stabilize the formulation of the cosmetic composition.

In one embodiment, agarose may be included in an amount of 0.7 to 2.0% by weight, specifically 1.0 to 2.0% by weight, and more specifically 1.0 to 1.5% by weight, based on the total weight of the oil-in-water cosmetic composition. When the agarose is included in an amount of less than 0.7% by weight, the stability over time of the formulation of the oil-in-water cosmetic composition may be somewhat reduced, and when the agarose is included in an amount exceeding 2.0% by weight, the melting temperature may increase, making it difficult to control flowability.

In this specification, the "melting temperature" refers to the temperature at which the gel begins to melt and become mushy, and when the melting temperature is low, it is easy to secure the flowability of the composition, so that the composition may be suitable for forming.

In the present invention, the auxiliary thickener may serve to improve the stability of the formulation and the sensory feel. Specifically, the auxiliary thickener may serve to reduce a water separation phenomenon and/or maintain the hardness of the oil-in-water cosmetic composition, thereby improving the stability over time of the oil-in-water cosmetic composition and thus improving marketability. The present inventors, in addition to agarose as an elastic thickener, derived an optimal combination of an auxiliary thickener, thereby achieving an oil-in-water cosmetic composition exhibiting excellent elasticity, improving processability by securing flowability, and also having excellent stability over time.

The "water separation phenomenon" refers to a phenomenon in which, as time passes, the network structure of a gel containing moisture contracts, releasing the moisture or other solvent contained inside the gel.

In one embodiment, the auxiliary thickener may include carrageenan and konjac root extract (Amorphophallus Konjac Root Extract). When both of these two components are included as the auxiliary thickener, an oil-in-water cosmetic composition having further excellent elasticity and/or hardness or the like may be obtained.

In one embodiment, carrageenan may be included in an amount of 0.05 to 0.4% by weight, specifically 0.05 to 0.3% by weight, and more specifically 0.2 to 0.3% by weight, based on the total weight of the oil-in-water cosmetic composition. When the carrageenan is included in an amount of less than 0.05% by weight, the water separation phenomenon may become somewhat more severe, increasing the amount of moisture lost over time, and when the carrageenan is included in an amount exceeding 0.4% by weight, elasticity or spreadability or the like may be reduced, resulting in a poor sensory feel.

In one embodiment, konjac root extract may be included in an amount of 0.05 to 0.15% by weight, specifically 0.05 to 0.12% by weight, and more specifically 0.07 to 0.12% by weight, based on the total weight of the oil-in-water cosmetic composition. When the konjac root extract is included in an amount of less than 0.05% by weight, the melting temperature may rise somewhat, reducing processability, and when the konjac root extract is included in an amount exceeding 0.15% by weight, the long-term stability of the formulation may be reduced.

In one embodiment, the oil-in-water cosmetic composition of the present invention may further include a UV-blocking agent. The UV-blocking agent may include any of an organic UV-blocking agent, an inorganic UV-blocking agent, and/or a UV-blocking agent in the form of an organic/inorganic composite, which can be employed by a person having ordinary skill in the art. Examples of the UV-blocking agent include at least one of Ethylhexyl Triazone, Diethylamino Hydroxybenzoyl Hexyl Benzoate, Bis-Ethylhexyloxyphenol Methoxyphenyl Triazine, Ethylhexyl Methoxycinnamate, Ethylhexyl Salicylate, Octocrylene, Diethylhexyl Butamido Triazone, Homosalate, Isoamyl p-Methoxycinnamate, Polysilicone-15, Methylene Bis-Benzotriazolyl Tetramethylbutylphenol, Tris-Biphenyl Triazine, Phenylbenzimidazole Sulfonic Acid, Terephthalylidene Dicamphor Sulfonic Acid, Titanium Dioxide, Bis-Ethylhexyloxyphenol Methoxyphenyl Triazine, and Zinc Oxide. However, the UV-blocking agent is not limited thereto, and various UV-blocking agents may be included.

In one embodiment, the oil-in-water cosmetic composition of the present invention may further include purified water, a polyol, an oil, a surfactant, a moisturizer, a preservative, or a functional ingredient.

In one embodiment, the oil may form the hardness of the stick-type cosmetic composition and provide sensory feel and a moisturized feel. Examples of the oil may include dibutyl adipate, octyldodecanol, caprylic/capric/succinic triglyceride, glyceryl caprylate, coco-caprylate/caprate, butyloctyl salicylate, and sunflower seed oil. However, the oil is not limited thereto.

In one embodiment, the powder may be used to adjust hardness or as a bulking agent. Examples of the powder may include silica, synthetic fluorphlogopite, methyl methacrylate crosspolymer, and the like. However, the powder is not limited thereto.

In one embodiment, the surfactant may serve to stabilize an emulsion formulation. Examples of the surfactant may include polyglyceryl-6 polyricinoleate, polyglyceryl-4 diisostearate/polyhydroxystearate/sebacate, cetyl PEG/PPG-10/1 dimethicone, and lauryl polyglyceryl-3 polydimethylsiloxyethyl dimethicone*tocopherol. However, the surfactant is not limited thereto.

In one embodiment, the moisturizer may provide moisture to the skin or maintain moisture of the skin, thereby providing a lasting moisturized feel. Examples of the moisturizer may include caprylyl glycol, butylene glycol, dipropylene glycol, methylpropanediol, pentylene glycol, glycereth-26, sorbitol, and diglycerin, but are not limited thereto.

In one embodiment, the preservative is used to enhance the effect of preservation, and for example, ethylhexylglycerin, 1,2-hexanediol, and the like may be used, but the preservative is not limited thereto.

In one embodiment, the functional ingredient may be added by mixing various ingredients depending on the purpose of the cosmetic composition, and may include, for example, all of a wrinkle improving agent, a whitening functional ingredient, a pigmentation functional ingredient, a skin-soothing functional ingredient, and a sebum or pore care functional ingredient.

In one embodiment, the oil-in-water cosmetic composition of the present invention may further include a colorant. The color of the oil-in-water cosmetic composition may be adjusted by appropriately blending the colorant according to the purpose of use.

In addition to the foregoing, the oil-in-water cosmetic composition of the present invention may further include ingredients commonly included in general cosmetic compositions, and may include, for example, a wax, a powder, an emulsion stabilizer, a skin conditioning agent, an ion sequestrant, a bactericide, a pH adjuster, an antioxidant, an alcohol, a plant extract, a fragrance, and a sensory feel modifier.

The blending amounts of the above-described ingredients are not particularly limited, and may be easily selected by a person skilled in the art within a range that does not impair the purpose and effects of the present invention.

In one embodiment, the oil-in-water cosmetic composition of the present invention may be provided in another formulation including an oil-in-water (O/W) type. For example, the oil-in-water cosmetic composition may be provided in a formulation such as a multiple emulsion formulation (O/W/O or W/O/W, etc.), a paste, or a foam.

In one embodiment, the oil-in-water cosmetic composition of the present invention may be provided in a stick-type. The oil-in-water cosmetic composition can form sufficient hardness and has excellent elasticity, has a low melting temperature so that flowability is easy to control, and also has excellent stability over time, and thus is easy to fill into a container for use, such as in a stick-type, and can maintain excellent quality over a long period of time. In addition, by being provided in the stick-type, the storage and use of the oil-in-water cosmetic composition may be easy. For example, the oil-in-water cosmetic composition may be manufactured into cosmetic products such as a sun stick, a sun balm, a lipstick, a lip balm, a lip gloss, a multi balm, a foundation, a lip cream, and a concealer.

In a specific embodiment, the oil-in-water cosmetic composition of the present invention may be a gel formulation having elasticity. As the gel formulation forms a gel network through hydrogen bonding and/or van der Waals forces of agarose, the gel formulation may have elasticity resulting from a restoring force of the gel network or the like. Accordingly, when the cosmetic composition is applied to the skin, the cosmetic composition may provide a user with a unique sensory feel due to elasticity, and may have a springy, jelly-like appearance, thereby causing consumers to feel an aesthetic appeal from the formulation.

In one embodiment, the oil-in-water cosmetic composition of the present invention may be manufactured into a semi-solid form through its unique elasticity and stability over time. By being manufactured into the semi-solid form, the oil-in-water cosmetic composition may have a hardness sufficient to maintain its shape, but the shape thereof may change according to pressure or a change in temperature.

In one embodiment, the oil-in-water cosmetic composition of the present invention may be a UV-blocking stick-type cosmetic composition. The oil-in-water cosmetic composition of the present invention may be provided, for example, in a form such as a sun stick or a sun balm.

In a specific embodiment, the oil-in-water cosmetic composition of the present invention may be provided in a semi-solid form included in a sun stick product. The sun stick product does not collapse during application, and can achieve a unique sensory feel due to elasticity, and due to its jelly-like appearance, may arouse an aesthetic curiosity or satisfaction in the user.

Hereinafter, examples and experimental examples for explaining the present invention in more detail are presented as follows, but the present invention is not limited thereto.

### Experimental Example 1. Comparative experiment according to combination of thickeners

### (1) Preparation of examples and comparative examples

In order to compare the effects according to whether or not a specific thickener was included, for the implementation of the oil-in-water cosmetic composition of the present invention, examples and comparative examples having a stick shape of a gel formulation were prepared as shown in Table 1 below. The amounts in the table below are in % by weight.

**[Table 1]**

| **Phase** | **Ingredient** | **Example** | **Comparative Example** | | | | | | |
|---|---|---|---|---|---|---|---|---|---|
| | | **1** | **1** | **2** | **3** | **4** | **5** | **6** | **7** |
| **A** | Agarose | 1 | 2 | 1.5 | 1 | 0.5 | 1 | 1 | - |
| | Carrageenan | 0.3 | - | - | - | - | 0.3 | - | 0.3 |
| | Konjac Root Extract | 0.1 | - | - | - | - | - | 0.1 | 0.1 |
| | Purified Water | To 100 | | | | | | | |
| **B** | UV-Blocking Agent | 10 | 10 | 10 | 10 | 10 | 10 | 10 | 10 |
| | Oil | 15 | 15 | 15 | 15 | 15 | 15 | 15 | 15 |
| | Moisturizer | 10 | 10 | 10 | 10 | 10 | 10 | 10 | 10 |
| | Formulation Stabilizer | 5 | 5 | 5 | 5 | 5 | 5 | 5 | 5 |

The specific preparation method is as follows.

The ingredients included in Phase A (water phase) were heated to 80°C or higher and then stirred to uniformly mix them, and the ingredients included in Phase B (oil phase) were heated to 90°C and then stirred to uniformly mix them. Phase B was slowly added to the uniformly stirred Phase A, and the mixture was uniformly emulsified at 95°C using a HOMOMIXER for 5 minutes. Thereafter, the emulsified mixture was filled into a rubber mold, cooled to room temperature, and the solidified content was applied to a stick container to complete the examples and comparative examples.

### (2) Evaluation of physical properties

Physical properties of the examples and comparative examples prepared with the ingredients and amounts as shown in Table 1 above were evaluated. Specifically, flowability, water separation rate, and stability of the formulation were evaluated.

For flowability, 100 g of each of the examples and comparative examples was taken and left to stand at 90°C for 30 minutes, and then the container was tilted to evaluate whether each of the examples and comparative examples had flowability. When no lumps formed in the sample after 30 minutes had elapsed, flowability was evaluated as "excellent," indicating that forming was easy and the melting temperature was low; when some lumps formed in the sample, flowability was evaluated as "moderate," indicating that forming was possible but a significant defect rate existed and the melting temperature was not low; and when many lumps formed in the sample, flowability was evaluated as "poor," indicating that forming itself was not easy and the melting temperature was high. When the duration of flowability was long, the melting temperature was low; when the melting temperature was moderate, the content flowed to a degree suitable for filling into a container; and when the duration of flowability was short, the melting temperature was high, and it was evaluated that flowability was insufficient, making it unsuitable for filling.

For the water separation rate, the examples and comparative examples were left to stand for 30 days, and the degree to which the water separation phenomenon occurred was measured and calculated according to the formula below.$\begin{matrix}\left(Water Separation Rate\right) = \\ \left[\frac{\left(Weight of Initial Composition\right) - \left(Weight of Composition After a Predetermined Time\right)}{\left(Weight of Initial Composition\right)}\times 100\left(%\right)\right]\end{matrix}$

When the water separation rate was 10% or less, it was evaluated as "low"; when the water separation rate exceeded 10% but was 15% or less, it was evaluated as "moderate"; and when it exceeded 15%, it was evaluated as "poor." A lower water separation rate corresponds to less moisture loss over time, and thus it was determined that a lower water separation rate indicates superior physical properties.

For the evaluation of formulation stability, the examples and comparative examples were left to stand at low temperature (5°C), room temperature (25°C), and high temperature (40°C) for 30 days, after which the degree of change in penetration value was measured, and the degree to which the appearance of the formulation was deformed was visually observed, together with whether the formulation collapsed when applied to the skin, and these were evaluated comprehensively. The penetration value was measured using a hardness tester with a 1 mm diameter pin under conditions of 30 cm/min and a depth of 10 mm.

The evaluation results according to the above experimental methods are shown in Table 2 below.

### (3) Sensory evaluation

A sensory evaluation was conducted on the examples and comparative examples prepared with the ingredients and amounts as shown in Table 1 above. Specifically, a panel of 10 professional evaluators applied each of the examples and comparative examples over the entire left arm, and then evaluated three items-elasticity, cooling sensation, and spreadability-on a 5-point scale. The closer the score for each item was to 5 points, the more excellent it was considered to be.

For elasticity, the evaluation focused on whether the elasticity of the formulation was sufficiently felt during application, such that the composition was felt to adhere strongly to the skin; for cooling sensation, the evaluation focused on whether a sufficient moist feel was felt at the applied area; and for spreadability, the evaluation focused on whether the composition was applied smoothly, without stiffness or a dragging sensation, during application.

The evaluation results according to the above evaluation methods are shown in Table 2 below, and a photograph of Example 1 is shown in FIG. 1.

**[Table 2]**

| **Item** | **Example** | **Comparative Example** | | | | | | |
|---|---|---|---|---|---|---|---|---|
| | **1** | **1** | **2** | **3** | **4** | **5** | **6** | **7** |
| Water Separation Rate | Low | Moderate | Moderate | Moderate | Poor | Low | Moderate | Low |
| Flowability | Low | High | High | Moderate | Low | Moderate | Low | Low |
| Formulation Stability | Excellent | Moderate | Moderate | Moderate | Poor | Moderate | Moderate | Poor |
| Elasticity | 5 | 5 | 5 | 5 | 1 | 5 | 5 | 0 |
| Cooling Sensation | 5 | 5 | 5 | 5 | 2 | 5 | 5 | 1 |
| Spreadability | 5 | 3 | 4 | 4 | 2 | 5 | 5 | 2 |

According to Table 2 above, Example 1, which included agarose as the elastic thickener and both carrageenan and konjac root extract as the auxiliary thickener in appropriate amounts, had a low water separation rate of 10% or less, and also had excellent formulation stability, confirming that the stability over time of the oil-in-water composition was excellent. In addition, it was confirmed that the melting temperature was low and the flowability was high, so that forming, such as filling into a container, was easy. Furthermore, as shown in the photograph of FIG. 1, the elasticity was excellent, thereby achieving a unique sensory feel, and it was confirmed that the spreadability was very excellent, resulting in an excellent sensory feel, and it was also confirmed that the excellent cooling sensation of the oil-in-water formulation was maintained. In contrast, according to Comparative Examples 1 to 4, which did not include the auxiliary thickener and included only agarose, it was confirmed that as the amount of the elastic thickener increased, elasticity improved and items such as the water separation rate gradually improved; however, as confirmed in Comparative Examples 1 and 2, flowability was very poor, to the extent that forming was not possible, and the formulation partially crumbled during application or the like, confirming that stability was also poorer than in the case where the auxiliary thickener was used. In the case of Comparative Examples 5 and 6, which did not include one of the two auxiliary thickeners, it was confirmed that, compared to the example, the penetration value greatly increased after storage, formulation stability was poor, and flowability was somewhat reduced or the water separation rate was high, resulting in reduced marketability. In particular, in the case of Comparative Example 7, which did not include agarose, it was confirmed that the formulation completely collapsed during application, to the extent that application was not possible, that formulation stability was very poor, that no elasticity was imparted at all, and that spreadability was very poor and cooling sensation was also not achieved.

Therefore, it was confirmed that including both a specific elastic thickener and an auxiliary thickener is an important component in the implementation of the oil-in-water cosmetic composition of the present invention.

### Experimental Example 2. Comparative experiment according to thickener content

### (1) Preparation of comparative examples

In order to confirm the optimal contents of the elastic thickener and the auxiliary thickener in the present invention, comparative examples were prepared as shown in Table 3 below, using the same preparation method as in Experimental Example 1 above. Specifically, based on Example 1, the content of any one of the three thickeners was varied, while the other ingredients and contents were kept the same. The unit in Table 3 is % by weight.

**[Table 3]**

| **Phase** | **Ingredient** | **Example** | **Comparative Example** | | | | | |
|---|---|---|---|---|---|---|---|---|
| | | **1** | **8** | **9** | **10** | **11** | **12** | **13** |
| | Agarose | 1 | 1 | 1 | 1 | 1 | 0.6 | 2.1 |
| **A** | Carrageenan | 0.3 | 0.3 | 0.3 | 0.02 | 0.5 | 0.3 | 0.3 |
| | Konjac Root Extract | 0.1 | 0.01 | 0.2 | 0.1 | 0.1 | 0.1 | 0.1 |

### (2) Evaluation of physical properties

Physical properties of the comparative examples prepared with the ingredients and amounts as shown in Table 3 above were evaluated by the same method as in Experimental Example 1 above, and the results are shown in Table 4 below.

### (3) Sensory evaluation

A sensory evaluation of the comparative examples prepared with the ingredients and amounts as shown in Table 3 above was conducted by the same method as in Experimental Example 1 above, and the results are shown in Table 4 below.

**[Table 4]**

| **Item** | **Comparative Example** | | | | | |
|---|---|---|---|---|---|---|
| | **8** | **9** | **10** | **11** | **12** | **13** |
| Water Separation Rate | Low | Low | Moderate | Low | Low | Low |
| Flowability | Moderate | Low | Low | Low | Low | High |
| Formulation Stability | Moderate | Moderate | Excellent | Excellent | Poor | Excellent |
| Elasticity | 5 | 5 | 5 | 3 | 1 | 5 |
| Cooling Sensation | 5 | 5 | 5 | 3 | 2 | 5 |
| Spreadability | 5 | 5 | 5 | 3 | 2 | 4 |

According to Table 4 above, Comparative Example 8, in which konjac root extract as the auxiliary thickener was included in an amount less than the appropriate amount, was confirmed to have somewhat poor flowability and formulation stability, and Comparative Example 9, in which konjac root extract was included in an amount greater than the appropriate amount, had poor formulation stability. Next, Comparative Example 10, in which carrageenan as the auxiliary thickener was included in an amount less than the appropriate amount, was confirmed to have a water separation phenomenon occurring more severely than in the example, resulting in decreased marketability over time, and in the case of Comparative Example 11, in which carrageenan was included in an excessive amount, it was confirmed that physical properties such as water separation rate and flowability were good, but elasticity and spreadability were somewhat reduced, and cooling sensation was also insufficient. Comparative Example 12, in which agarose was included in an amount less than the appropriate amount, was confirmed to have the formulation deteriorate and collapse, resulting in very poor formulation stability, and received very low scores in all three items of the sensory evaluation, confirming that elasticity was markedly insufficient; and Comparative Example 13, in which agarose was included in an amount exceeding the appropriate amount, was confirmed to have poor flowability, making forming difficult, and spreadability was slightly insufficient.

Therefore, it was confirmed that including a specific elastic thickener and auxiliary thickener in appropriate amounts is an important component in the implementation of the oil-in-water cosmetic composition of the present invention.

While specific aspects of the content of the present invention have been described in detail above, it will be apparent to a person having ordinary skill in the art that these specific descriptions are merely preferred embodiments, and the scope of the present invention is not limited thereby. Therefore, the substantial scope of the present invention will be defined by the appended claims and their equivalents.

## Claims

1. An oil-in-water (O/W) cosmetic composition comprising an elastic thickener and an auxiliary thickener, wherein the elastic thickener comprises agarose.

2. The oil-in-water cosmetic composition of claim 1, wherein the auxiliary thickener comprises carrageenan and konjac root extract (Amorphophallus Konjac Root Extract).

3. The oil-in-water cosmetic composition of claim 1, wherein the agarose is included in an amount of 0.7 to 2.0% by weight based on a total weight of the oil-in-water cosmetic composition.

4. The oil-in-water cosmetic composition of claim 2, wherein the carrageenan is included in an amount of 0.05 to 0.4% by weight based on a total weight of the oil-in-water cosmetic composition.

5. The oil-in-water cosmetic composition of claim 2, wherein the konjac root extract is included in an amount of 0.05 to 0.15% by weight based on a total weight of the oil-in-water cosmetic composition.

6. The oil-in-water cosmetic composition of claim 1, wherein the oil-in-water cosmetic composition further comprises a UV-blocking agent.

7. The oil-in-water cosmetic composition of claim 1, wherein the oil-in-water cosmetic composition is semi-solid.

8. The oil-in-water cosmetic composition of claim 1, wherein the oil-in-water cosmetic composition is a gel formulation having elasticity.

9. The oil-in-water cosmetic composition of claim 1, wherein the oil-in-water cosmetic composition is a stick-type cosmetic composition.
